# EUROPEAN PATENT APPLICATION

(11) **EP 4 699 634 A1**
(43) Date of publication of application: **25.02.2026**
(21) Application number: 24795624.6
(22) Date of filing: 08.03.2024
(51) Int. Cl.: A61M 16/00, A61M 16/16, A61M 16/10, F16B 1/04

(54) **PUSH BUTTON, WATER TANK, AND VENTILATION TREATMENT DEVICE**

(30) Priority: 25.04.2023 WO PCT/CN2023/090623
(71) Applicant: BMC Medical Co., Ltd., Beijing 100142 (CN); BMC (Tianjin) Medical Co., Ltd., Tianjin 301700 (CN)
(72) Inventor: ZHI, Jianxin, Beijing 100142 (CN); LIU, Lijun, Beijing 100142 (CN); ZHANG, Jie, Beijing 100142 (CN); ZHANG, Jiayin, Beijing 100142 (CN); GUO, Xiaoshuai, Tianjin 301700 (CN); ZHUANG, Zhi, Beijing 100142 (CN)
(74) Representative: Grünecker Patent- und Rechtsanwälte PartG mbB
(86) International application number: PCT/CN2024/080803
(87) International publication number: WO 2024/222238

(57) **Abstract**

Embodiments of this specification provide a press button for mounting and detachment of a water tank from a main unit of a ventilation therapy device. The press button is arranged on the water tank, and is configured to be pressed toward the interior of a water tank housing of the water tank under the action of an external force such that the water tank is mounted to or detached from the main unit. The press button includes a pressing body, the pressing body including a resilient structure, the resilient structure including a fixed end and a free end arranged opposite each other, and the resilient structure being configured such that: when the press button is pressed toward the interior of the water tank housing, the free end moves from a first position to a second position, and the resilient structure elastically deforms; and after the external force is released, the free end moves from the second position to the first position, and the resilient structure restores its shape to drive the press button to resiliently return to its original position away from the interior of the water tank housing such that the water tank is connected to the main unit or returns to its original position after detachment. The embodiments of this specification further provide a water tank including a press button, and a ventilation therapy device including the above water tank.

## Description

### Cross-References

This specification claims priority to the international application No. PCT/CN2023/090623 filed on April 25, 2023, which is incorporated herein by reference in its entirety.

### Technical Field

This specification relates to the technical field of medical devices, and in particular to a press button, a water tank and a ventilation therapy device.

### Background Art

Ventilation therapy devices (such as a ventilator), as devices with artificial ventilation, have been widely used in the treatment of diseases such as respiratory failure and sleep apnea hypopnea syndrome caused by various reasons, and are extremely important in the field of modern medicine. When a user uses a ventilation therapy device for the treatment of a respiratory disease, the ventilation therapy device needs to draw in gas from the outside and perform a series of gas treatments before the gas can be supplied to the user for breathing. Current ventilation therapy devices often have many problems. For example, it is inconvenient for mounting and detachment of a water tank, which is used for humidifying respiratory gas supplied to a user, on a ventilation therapy device from a main unit of the ventilation therapy device.

In order to solve the above problem, this specification aims to provide a press button, a water tank and a ventilation therapy device.

### Summary

One of embodiments of this specification provides a press button for mounting and detachment of a water tank from a main unit of a ventilation therapy device. The press button is arranged on the water tank, and is configured to be pressed toward an interior of a water tank housing of the water tank under the action of an external force such that the water tank is mounted to or detached from the main unit; The press button includes a pressing body, the pressing body including a resilient structure, the resilient structure including a fixed end and a free end arranged opposite the fixed end, and the resilient structure being configured such that: when the press button is pressed toward the interior of the water tank housing, the free end moves from a first position to a second position, and the resilient structure elastically deforms; and after the external force is released, the free end moves from the second position to the first position, and the resilient structure restores its shape to drive the press button to resiliently return to its original position away from the interior of the water tank housing such that the water tank is connected to the main unit or returns to its original position after detachment.

One of embodiments of this specification provides another press button for mounting and detachment of a water tank from a main unit of a ventilation therapy device. the press button is arranged on the water tank, and is configured to be pressed toward an interior of a water tank housing of the water tank under the action of an external force such that the water tank is mounted to or detached from the main unit; The press button includes a pressing body, the pressing body including a resilient structure, the resilient structure including an elastic arm, the elastic arm including a free end and a fixed end arranged opposite the free end, and the elastic arm being configured such that: when the press button is pressed toward the interior of the water tank housing, the free end slides away from the fixed end, and the elastic arm elastically deforms; and after the external force is released, the free end slides toward the fixed end, and the elastic arm restores its shape such that the water tank is connected to the main unit or returns to its original position after detachment.

One of embodiments of this specification provides another press button for mounting and detachment of a water tank from a main unit of a ventilation therapy device. the press button is arranged on the water tank, and is configured to be pressed toward an interior of a water tank housing of the water tank under the action of an external force such that the water tank is mounted to or detached from the main unit; The press button includes a pressing body, the pressing body includes a resilient structure, the resilient structure includes an elastic member, and the elastic member is configured such that: when the press button is pressed toward the interior of the water tank housing, the elastic member has a decrease in height and elastically deforms; and after the external force is released, the elastic member has an increase in height and restores its shape such that the water tank is connected to the main unit or returns to its original position after detachment.

One of the embodiments of this specification provides a water tank including a press button as described above in any one of the embodiments.

One of the embodiments of this specification provides a ventilation therapy device including a water tank as described above.

The embodiments of this specification may bring beneficial effects including but not limited to: (1) the arrangement of the press button on the water tank enables the detachable connection between the water tank and the main unit of the ventilation therapy device, which reduces the operation difficulty of the detachment of the water tank and allows the water tank to be connected to the main unit in a flexible manner; (2) the arrangement of the resilient structure enables the press button to automatically return to its original position after being pressed, which does not require additional operations and has simple steps; and (3) the movable arrangement of the press button relative to the water tank housing enables the water tank to remain stable during the detachment process and be not prone to shaking relative to the main unit, which not only increases the diversity of connection between the water tank and the main unit, but also reduces the difficulty of connecting the water tank to the main unit, making it easier for the mounting and detachment of the water tank.

### Brief Description of the Drawings

This specification will be further illustrated by way of exemplary embodiments, and these exemplary embodiments will be described in detail with reference to the drawings. These embodiments are not restrictive, and in these embodiments, the same numerals denote the same structures. In the drawings:

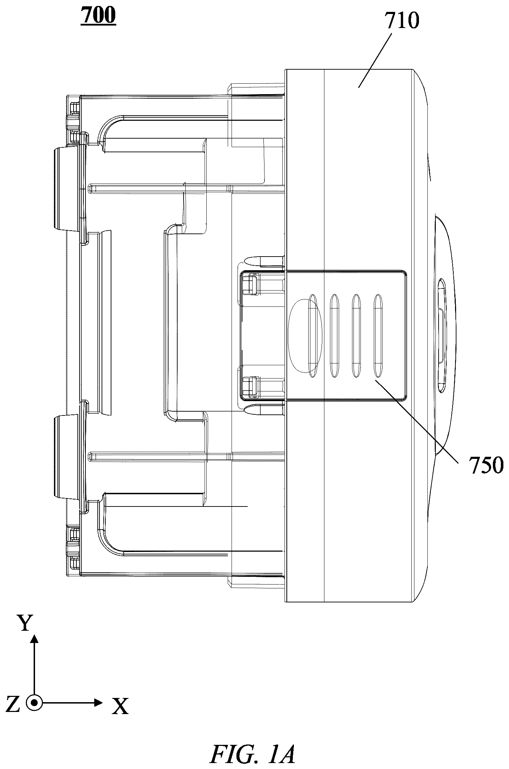

FIG. 1A is an exemplary schematic diagram of an appearance of a water tank according to some other embodiments of this specification;

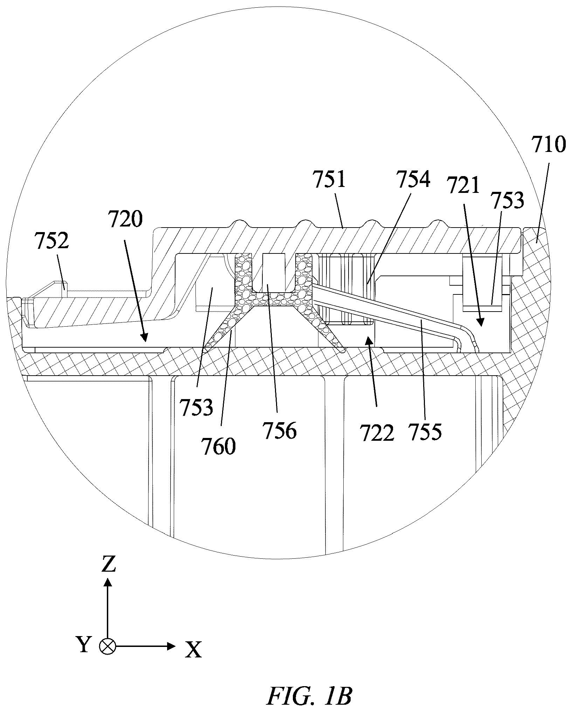

FIG. 1B is an exemplary schematic diagram of an mating structure of a press button and a recessed structure according to some embodiments of this specification;

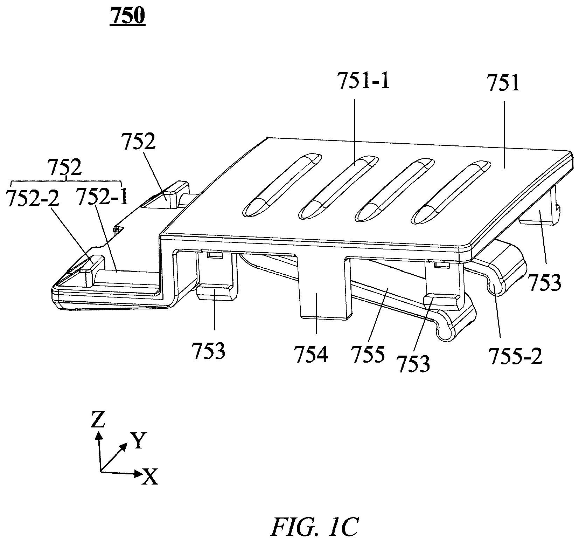

FIG. 1C is an exemplary structural schematic diagram of the press button according to some embodiments of this specification;

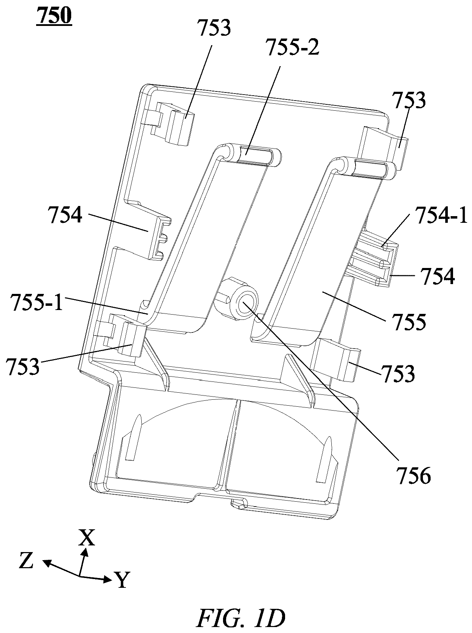

FIG. 1D is an exemplary structural schematic diagram of the press button from another perspective according to some embodiments of this specification;

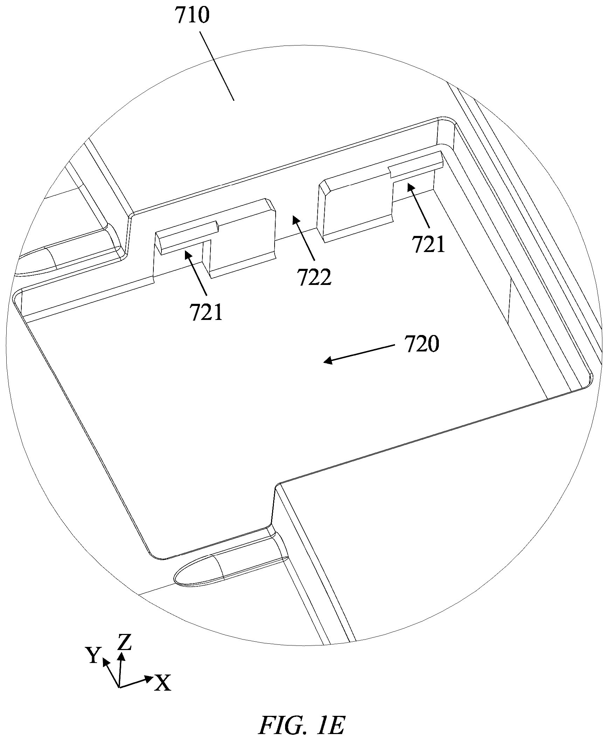

FIG. 1E is an exemplary structural schematic diagram of the recessed structure according to some embodiments of this specification; and

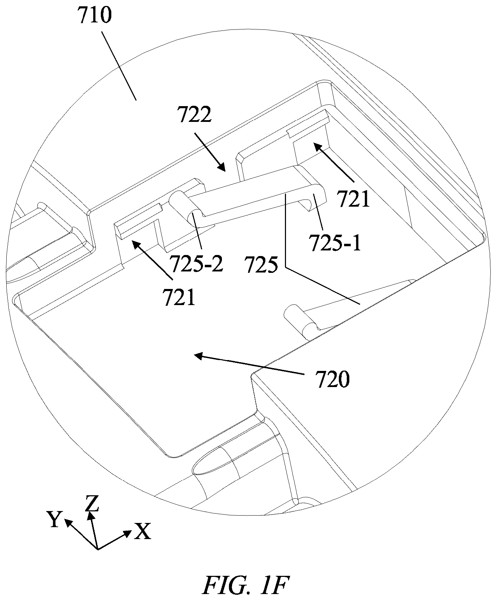

FIG. 1F is another exemplary structural schematic diagram of the recessed structure according to some embodiments of this specification; and

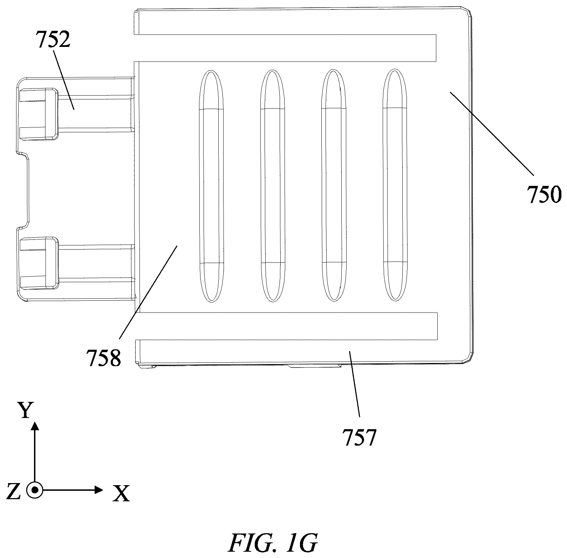

FIG. 1G is another exemplary structural schematic diagram of the press button according to some embodiments of this specification.

List of reference signs: 700. Water tank; 710. Water tank housing; 720. Recessed structure; 721. Limiting slot; 722. guide groove; 725. Second elastic arm; 725-1. Second fixed end; 725-2. Second free end; 750. Press button; 751. Pressing surface; 751-1. Pressing stripe; 752. Connecting structure; 752-1. Connecting member; 752-2. Snap-fit protrusion; 753. Limiting member; 754. Guide member; 754-1. Reinforcing protrusion; 755. First elastic arm; 755-1. First fixed end; 755-2. First free end; 756. Connecting protrusion; 757. Fixing structure; 758. Elastic piece mechanism; 760. Elastic member.

### Detailed Description of Embodiments

Exemplary embodiments or implementations are described in detail herein, with examples shown in the accompanying drawings. Unless otherwise indicated, the same numbers in different accompanying drawings represent the same or similar elements when the following description refers to the accompanying drawings. The implementations described in the following exemplary embodiments do not represent all implementations consistent with the embodiments of the present application. Instead, the implementations are merely examples of apparatuses and methods consistent with some aspects of the present application as detailed in the appended claims.

Terms used in the present application are merely for the purpose of describing specific embodiments, but are not intended to limit the present application. Singular forms "a/an", "said" and "the" used in the present application and the appended claims are also intended to include plural forms unless otherwise clearly indicated in the context.

It should be understood that the terms "first", "second" and the like used in the specification of the present application and the claims do not denote any order, quantity or importance, but are only used to distinguish different components. Similarly, the words "a" or "an" and the like do not indicate a quantitative limit, but rather indicate the presence of at least one. Unless otherwise indicated, terms such as "front", "rear", "lower" and/or "upper" are used for ease of description only and are not intended to be limited to one position or one spatial orientation. The term such as "comprise" or "include" means that the element or object appearing before "comprise" or "include" encompasses the elements or objects and equivalents listed after "comprise" or "include", and does not exclude other elements or objects.

In modern clinical medicine, ventilation therapy devices (e.g., a ventilator), as effective means to artificially replace the spontaneous ventilation function, are apparatuses that can replace, control or change a person's normal physiological breathing, increase lung ventilation, improve the respiratory function, and reduce the consumption of work of breathing, have been widely used in respiratory failure caused by various reasons, and anesthesia respiratory management, respiratory support therapy and emergency resuscitation during major surgery, and are extremely important in the field of modern medicine. The ventilation therapy devices are vital medical devices that can function to prevent and treat respiratory failure, reduce complications, and save and extend patients' lives. The ventilation therapy devices available in the market have problems such as difficult operations and laborious detachment during the mounting and detachment of a water tank from a main unit.

FIG. 1A is an exemplary schematic diagram of an appearance of a water tank according to some other embodiments of this specification; FIG. 1B is a exemplary schematic diagram of an mating structure of a press button and a recessed structure according to some embodiments of this specification; FIG. 1C is an exemplary structural schematic diagram of the press button according to some embodiments of this specification; FIG. 1D is an exemplary structural schematic diagram of the press button from another perspective according to some embodiments of this specification; FIG. 1E is an exemplary structural schematic diagram of the recessed structure according to some embodiments of this specification; and FIG. 1F is another exemplary structural schematic diagram of the recessed structure according to some embodiments of this specification. The structure of a water tank 700 is described below with reference to FIGS. 1A to 1F.

As shown in FIG. 1A, some embodiments of this specification provide a water tank 700 including a press button 750. The press button 750 can be used for mounting and detachment of the water tank 700 from a main unit in a ventilation therapy device. In some embodiments, the water tank 700 includes a water tank housing 710, and the press button 750 is arranged on an outer surface of the water tank housing 710. Referring to FIGS. 1A and 1B, the press button 750 is configured to be pressed toward the interior of the water tank housing 710 under the action of an external force to enable the water tank 700 to be mounted to or detached from the main unit. In some embodiments, the press button 750 includes a pressing body. The pressing body includes a resilient structure (not shown). The resilient structure can be configured such that: when the press button 750 is pressed toward the interior of the water tank housing 710, the resilient structure elastically deforms; and after the external force is released, the resilient structure restores its shape and drives the press button 750 to resiliently return to its original position away from the interior of the water tank housing 710 to enable the water tank 700 to be connected to the main unit or return to its original position after detachment. Therefore, during the mounting or release process of the water tank 700, the whole water tank 700 can remain stable (e.g., the relative positions of parts of the water tank housing 710 remain unchanged, etc.), which facilitates the connection between the water tank 700 and the main unit and reduces the difficulty of connection.

In some embodiments, the resilient structure includes a fixed end and a free end, the fixed end being arranged opposite the free end. The resilient structure is configured such that: when the press button 750 is pressed toward the interior of the water tank housing 710, the free end moves from a first position to a second position, and the resilient structure elastically deforms; and after the external force is released, the free end moves from the second position to the first position, and the resilient structure restores its shape to drive the press button 750 to resiliently return to its original position away from the interior of the water tank housing 710 to enable the water tank 700 to be connected to the main unit or return to its original position after being detached from the main unit. In this embodiment, due to the elastic deformation of the resilient structure, at least part of the structure of the press button 750 has a space of movement relative to the water tank housing 710, and the movement of the at least part of the structure of the press button 750 enables the water tank 700 to be connected to or separated from a corresponding engagement portion of the main unit. In this embodiment, due to the movement of the free end of the resilient structure between the first position and the second position, the at least part of the structure of the press button 750 has a space of movement. In actual applications, the above movable part of the press button 750 may move in a small range of space of movement, for example, move within a range from 0.1 mm to 1 mm, and of course, may also move within a range of less than 0.1 mm or greater than 1 mm, depending on the structural size in the actual application. However, for the stability of the pressing operation of the press button 750, a maximum pressing range is preferably not more than 15 mm.

In some embodiments, the press button 750 may be arranged on an upper surface of the water tank housing 710, and the press button 750 may be pressed downward under the action of an external force; and after the external force is released, the press button 750 may be driven by the resilient structure to resiliently return upward to its original position. In some embodiments, the press button 750 may also be arranged on a bottom surface, a front surface, a side surface, or other surfaces of the water tank housing 710. In some embodiments, one or more press buttons 750 may be provided. For example, one press button 750 may be provided and arranged on the upper surface, the front surface, etc. of the water tank housing 710. For another example, two press buttons 750 may be provided and arranged on different surfaces of the water tank housing 710, respectively. Considering the operability of the actual mounting and detachment of the water tank 700 from the main unit, if there are too many press buttons 750, it will be difficult for an operator to operate and to operate with one hand. Therefore, one or two press buttons 750 may be provided. Moreover, when two press buttons 750 are provided, the two press buttons 750 are respectively arranged on opposite surfaces, such as the upper surface and the bottom surface, of the water tank housing 710. For ease of description, the structure of the press button 750 and the water tank housing 710 is described below with respect to an example in which the press button 750 is arranged on the upper surface of the water tank housing 710. It should be noted that the structure of the press button 750 arranged on other surfaces of the water tank housing 710 is the same as or similar to the structure of the press button 750 arranged on the upper surface of the water tank housing 710, and will not be repeated in this specification.

In some embodiments, a length direction of the water tank housing 710 may be defined as an X direction, a width direction of the water tank housing 710 may be defined as a Y direction, and a height direction of the water tank housing 710 may be defined as a Z direction. And, a rightward direction is the X direction, and a leftward direction is an direction opposite the X direction. The water tank housing 710 moves to the left (i.e., in the direction opposite the X direction) to enter the main unit to achieve connection. An upward direction is the Z direction, and a downward direction is a direction opposite the Z direction. The press button 750 moves downward, that is, the press button 750 moves in the direction opposite the Z direction; The press button 750 moves upward, that is, the press button 750 moves in the Z direction. A forward direction is the direction opposite the Y direction, and a rearward direction is the Y direction.

In some embodiments, the press button 750 further includes a connecting structure 752. The connecting structure 752 is detachably connected to the main unit. The pressing body includes a pressing structure, and the connecting structure 752 is arranged at an end of the pressing body close to the main unit, that is, the connecting structure 752 is arranged at an end of the pressing structure close to the main unit. In the case where the press button 750 is arranged on the upper surface, that is, the top surface, of the water tank housing 710, when the press button 750 is pressed toward the interior of the water tank housing 710, that is, applies a force to the upper surface (i.e., a pressing surface 751) of the pressing structure toward the interior of the water tank housing 710, that is, presses the pressing structure downward, the connecting structure 752 moves toward the interior of the water tank housing 710 synchronously with and in the same direction as the pressing structure, that is, the connecting structure 752 moves downward to cause the connecting structure 752 to be disengaged from the corresponding engagement portion of the main unit to separate the connecting structure 752 from the main unit, so that the water tank 700 can be detached from the main unit. After the press button 750 returns to its original position, the connecting structure 752 may be connected to the main unit, so as to achieve a fixed connection between the water tank 700 and the main unit.

As shown in FIGS. 1B and 1C, in some embodiments, the connecting structure 752 is arranged at the end of the press button 750 close to the main unit, that is, the connecting structure 752 is arranged on a left side of the pressing body in the X direction, to facilitate the connection between the connecting structure 752 and the main unit. In some embodiments, the connecting structure 752 may include a connecting member 752-1 and a snap-fit protrusion 752-2. The connecting member 752-1 and the snap-fit protrusion 752-2 (or groove) which is configured for connection with the main unit are arranged on the upper surface of the connecting structure 752 (i.e., away from the water tank housing 710). The connecting member 752-1 is connected to the end of the pressing body, and the snap-fit protrusion 752-2 is arranged at an end of the connecting member 752-1 away from the pressing body. Correspondingly, an inner surface of the housing of the main unit, particularly an inner surface of the top housing of the main unit in this embodiment, provides an engagement portion corresponding to the connecting structure 752, and a groove (or protrusion) that engages with the snap-fit protrusion 752-2 is provided at the engagement portion. Since the connecting member 752-1 has a preset length, for example, a length ranging from 5 mm to 25 mm, the engagement portion of the main unit and the groove should be arranged at a position corresponding to the preset length of the connecting member 752-1. The snap-fit protrusion 752-2 has only a freedom of movement relative to the groove in the direction of movement of the movable part of the press button 750 (e.g., in the Z direction, that is, the upward and downward directions). For example, when the pressing structure of the press button 750 is pressed downward, the connecting structure 752 moves downward synchronously, and in this case, the connecting structure 752 is located below and at a distance, such as 0.05 mm or 0.3 mm, from the engagement portion of the main unit. During the mounting of the water tank 700, when the pressing structure is pressed toward the interior of the water tank housing 710 (e.g., downward) under the action of an external force, the snap-fit protrusion 752-2 synchronously moves toward the interior of the water tank housing 710 (e.g., downward), and in this case, the water tank 700 may be moved to the left (i.e., in the direction opposite the X direction) to enter the main unit until the snap-fit protrusion 752-2 moves to a corresponding position below the groove. After the external force is released, the pressing structure resiliently return to its original position away from the interior of the water tank housing 710 (e.g., upward), and the snap-fit protrusion 752-2 enters the groove to achieve snap-fit connection and fixation, so as to lock the connection between the water tank 700 and the main unit. During the mounting and detachment of the water tank 700, when the pressing structure is pressed toward the interior of the water tank housing 710 (e.g., downward) under the action of the external force, the snap-fit protrusion 752-2 moves toward the interior of the water tank housing 710 (e.g., downward) to exit the groove, so as to unlock the connection between the water tank 700 and the main unit. In this case, the water tank 700 may be moved to the right (i.e., in the X direction) to exit the main unit, so as to separate the water tank 700 from the main unit. After the water tank 700 is detached, the external force may be released, and the press button 750 may return to its original position.

In some embodiments, the connecting structure 752 and the main unit may also be connected in such other ways that the connection can be locked or unlocked by means of movement. For example, the connecting structure 752 and the main unit may be magnetically connected. When the press button 750 moves away from the interior of the water tank housing 710 (e.g., upward, i.e., in the Z direction) until the connecting structure 752 comes into contact with and is magnetically connected to the main unit, the connection between the water tank 700 and the main unit is locked; and when the press button 750 moves toward the interior of the water tank housing 710 (e.g., downward, i.e., in the direction opposite the Z direction) until the connecting structure 752 is separated from the main unit and the connecting structure 752 does not automatically approach the main unit under the action of a magnetic force, the connection between the water tank 700 and the main unit can be unlocked.

Referring to FIGS. 1B, 1E and 1F, in some embodiments, the upper surface of the water tank housing 710 includes a recessed structure 720, and the press button 750 is received in the recessed structure 720. The press button 750 may be moved in the recessed structure 720 toward or away from the interior of the water tank housing 710 (e.g., upward or downward), so as to lock or unlock the connection between the water tank 700 and the main unit. The resilient structure is arranged between a side of the press button 750 close to the interior of the water tank housing 710 (i.e., a lower side) and a bottom wall of the recessed structure 720. During the process of restoring its shape, the resilient structure can drive the press button 750 to move away from the bottom wall of the recessed structure 720 (i.e., away from the interior of the water tank housing 710, i.e., upward).

Referring to FIGS. 1B and 1C, in some embodiments, the free end of the resilient structure is configured to abut against the surface of the water tank housing 710, and the fixed end is fixedly connected to the pressing structure. The upper surface of the pressing structure serves as a pressing surface 751. The pressing surface 751 is arranged on a surface of the press button 750 away from the interior of the water tank housing 710 (i.e., the upper surface of the pressing structure) to provide a region for the user to press. Therefore, the resilient structure is arranged on a lower surface of the pressing structure, the fixed end of the resilient structure is connected to the lower surface of the pressing structure, and the free end of the resilient structure abuts against a top surface of the water tank housing 710. In some embodiments, the connecting structure 752 may be integrally formed with the pressing structure, or the connecting structure 752 may be integrally formed with the pressing structure and the resilient structure, which reduces the steps and difficulty of manufacturing the press button 750 and thus enables the connecting structure 752 to move synchronously with and in the same direction as the pressing structure or the pressing surface 751. In some embodiments, the pressing structure is arranged between the free end and the fixed end of the resilient structure, and applying a force to the pressing structure can change the relative positions of the free end and the fixed end. For example, when the force is applied to the pressing structure, the free end gradually moves toward the fixed end, and an included angle between the free end and the fixed end in the horizontal direction gradually increases, that is, the distance between the free end and the fixed end in the horizontal direction decreases.

Referring to FIG. 1C, in some embodiments, pressing stripes 751-1 are provided on the pressing surface 751. The plurality of pressing stripes 751-1 may be spaced apart in the X direction. The pressing stripes 751-1 may serve as a touch prompt, prompting the user where to press on the pressing surface 751. In addition, the pressing stripes 751-1 can also have the functions of increased frictions and slip resistance on the pressing surface 751 during operations by the user, thereby improving the operation safety of the water tank 700.

Referring to FIGS. 1B, 1C and 1D, in some embodiments, at least one of a limiting structure and a guide structure is further provided between the press button 750 and an outer surface of the water tank housing 710 (e.g., the recessed structure 720). The limiting structure may be configured to limit the range of movement of the press button 750 away from the interior of the water tank housing 710 (e.g., upward), and the press button 750 may be connected to the recessed structure 720 by means of the limiting structure. The guide structure may be configured to guide the press button 750 to move along a preset path.

In some embodiments, two or more limiting structures may be provided. In some embodiments, limiting structures may be provided on two sides (a front side and a rear side) of the press button 750 in the width direction (i.e., the Y direction), and the number of limiting structures on the two sides may be the same or different. For example, two limiting structures may be provided on each of the two sides of the press button 750 in the Y direction. The two limiting structures on either side are spaced apart in the X direction to enhance the connection strength between the press button 750 and the recessed structure 720. In some embodiments, one or more limiting structures may also be provided on the right side of the press button 750 in the X direction. In some embodiments, limiting structures may be provided on one, two, or three of the front, rear, and right sides of the press button 750, and the number of limiting structures provided on each side may be the same or different.

In some embodiments, the limiting structures may include limiting members 753 and limiting slots 721. The limiting members 753 may be arranged on one of the side of the press button 750 close to the interior of the water tank housing 710 (i.e., the lower side) or the outer surface of the water tank housing 710 (e.g., the recessed structure 720), and the limiting slots 721 may be arranged on the other of the side of the press button 750 close to the interior of the water tank housing 710 (i.e., the lower side) or the outer surface of the water tank housing 710 (e.g., the recessed structure 720). For example, as shown in FIGS. 1E and 1F, the limiting members 753 may be arranged on the side of the press button 750 close to the interior of the water tank housing 710 (i.e., the lower side), and the limiting slots 721 may be arranged in the recessed structure 720. The limiting slots 721 may fit with the limiting members 753 to provide a limiting function during the process of the press button 750 returning to its original position, so as to limit the press button 750 in the recessed structure 720. In some embodiments, the limiting members 753 are located in the limiting slots 721, and the limiting members 753 may move in the limiting slots 721 toward and/or away from the interior of the water tank housing 710 (e.g., the upward and downward directions). The height of the limiting slots 721 is the range of movement of the press button 750. In some embodiments, the limiting slots 721 are arranged in an inner wall of the recessed structure 720 and in one-to-one correspondence with the limiting members 753. That is, the limiting slots 721 are in one-to-one correspondence with the limiting members 753 in terms of position and number. For example, four limiting structures are provided between the press button 750 and the recessed structure 720. Two of the limiting structures are spaced apart in the X direction on the front side of the press button 750 in the Y direction, and the other two of the limiting structures are spaced apart in the X direction on a rear side of the press button 750 in the Y direction. In this case, two limiting members 753 are spaced apart in the X direction on the front side and the rear side of the press button 750 in the Y direction, respectively; and correspondingly, two inner side walls of the recessed structure 720 in the Y direction may each be provided with two limiting slots 721 spaced apart in the X direction. In some embodiments, the limiting member 753 may include but is not limited to a catch, a hook, a stop block, or other structures that can fit with the limiting slot 721.

In some embodiments, the limiting slot 721 has a preset height in the Z direction, so that when the limiting member 753 moves in the limiting slot 721 to come into contact with the inner wall of the end of the limiting slot 721 in the Z direction, the limiting member 753 is engaged in the limiting slot 721 and is engaged and limited to an end edge of the limiting slot 721, and in this case, the pressing surface 751 of the press button 750 may be flush with the outer surface (e.g., the upper surface) of the water tank housing 710, as shown in FIG. 1B, so as to prevent the pressing surface 751 from protruding or being recessed relative to the outer surface of the water tank housing 710 at the recessed structure 720, thereby preventing the pressing surface 751 from affecting the connection between the water tank 700 and the main unit.

In some embodiments, the guide structure includes a guide member 754 and a guide groove 722. The guide member 754 may be arranged on one of the side of the press button 750 close to the interior of the water tank housing 710 (i.e., the lower side) or the outer surface of the water tank housing 710 (e.g., the recessed structure 720), and the guide groove 722 may be arranged on the other of the side of the press button 750 close to the interior of the water tank housing 710 (i.e., the lower side) or the outer surface of the water tank housing 710 (e.g., the recessed structure 720). For example, as shown in FIGS. 1B, 1C and 1D, the guide member 754 may be arranged on a side of the pressing surface 751 close to the interior of the water tank housing 710 (e.g., the lower side), the guide groove 722 may be arranged in the recessed structure 720, and the guide member 754 can move in the guide groove 722 toward and/or away from the interior of the water tank housing 710 (e.g., the upward and downward directions). The guide member 754 may fit with the guide groove 722, and the guide groove 722 may serve as a preset path for guiding the direction of movement of the press button 750 during the process of being pressed and resiliently returning to its original position, so that the guide member 754 moves within the guide groove 722 during the movement of the press button 750, which prevents the press button 750 from tilting in the X direction and/or Y direction during the movement, so that the pressing surface 751 can maintain stable in the X direction and/or Y direction, thereby improving the stability of the press button 750.

In some embodiments, the guide structure may be arranged in an estimated stressed point region of the pressing surface 751 (e.g., in the vicinity of a middle portion in the length direction). The above arrangement can minimize a distance between the force position on the pressing surface 751 and the guide structure in the X direction, so that when the user presses the pressing surface 751, a force direction on the press button 750 may substantially coincide with an extension direction (i.e., the Z direction or the direction opposite the Z direction) of the guide structure (e.g., the guide member 754 and/or the guide groove 722), thereby reducing the frictions during the movement of the press button 750 and reducing the difficulty of pressing. Therefore, in order to effectively and smoothly drive the connecting structure 752 to move, the pressing region on the press button 750 that is acted upon by an external force is preferably located in the vicinity of the middle region thereof. A position in the vicinity of the middle region of the press button 750 in the length direction refers to a position in the vicinity of the middle of the other parts than the connecting structure 752 in the length direction, that is, in the vicinity of the middle region of the push button in the length direction of the pressing body. In some embodiments, the pressing region mentioned above is preferably located in the vicinity of the middle region of the pressing structure in the length direction. In addition, generally, a stressed center of the resilient structure is also correspondingly located in the vicinity of the middle position of the pressing structure, that is, a perpendicular at the stressed center of the resilient structure substantially coincides with a perpendicular at the middle portion of the pressing structure.

Referring to FIG. 1D, in some embodiments, a reinforcing protrusion 754-1 may be further provided on a side of the guide member 754 away from the inner wall of the recessed structure 720, and the reinforcing protrusion 754-1 may extend in the Z direction. The reinforcing protrusion 754-1 can enhance the strength of the guide member 754 and reduce the possibility of the guide member 754 being broken or deformed by a force. In some embodiments, the guide member 754 may be provided with a plurality of reinforcing protrusions 754-1, and the plurality of reinforcing protrusions 754-1 are spaced apart in the X direction.

Referring to FIG. 1D, in some embodiments, a guide structure may be provided, between the press button 750 and the recessed structure 720, on each of the two sides (the front side and the rear side) of the press button 750 in the Y direction. The two guide structures jointly guide the movement of the press button 750, improving the stability of the press button 750. In some embodiments, in the X direction, the guide structure (e.g., the guide member 754 and/or the guide groove 722) may be located between two limiting structures (e.g., the limiting member 753 and/or the limiting slot 721), as shown in FIG. 1D.

Referring to FIGS. 1E and 1F, in some embodiments, the guide grooves 722 may be in one-to-one correspondence with the guide members 754 in terms of position and number. For example, two guide structures are provided between the press button 750 and the recessed structure 720. One of the guide structures is arranged on the front side of the press button 750 in the Y direction, and the other is arranged on the rear side of the press button 750 in the Y direction. In this case, a guide member 754 may be provided on each of the two sides (the front side and the rear side) of the press button 750 in the Y direction, and the guide member 754 on each side is arranged at the estimated stressed point region of the pressing surface 751 (e.g., in the vicinity of the middle portion of the length direction). Correspondingly, a corresponding guide groove 722 may be provided on each of the two inner side walls of the recessed structure 720 in the Y direction, and the guide groove 722 on each side may be arranged at a position corresponding to the middle portion of the pressing surface 751.

Referring to FIGS. 1B to 1D, in some embodiments, the resilient structure may include at least one of an elastic arm (e.g., a first elastic arm 755 and/or a second elastic arm 725) and an elastic member 760. The elastic arm includes a free end and a fixed end, and the elastic arm is configured such that: when the press button 750 is pressed toward the interior of the water tank housing 710 (e.g., downward), that is, when the pressing structure and the connecting structure 752 are synchronously pressed toward the interior of the water tank housing 710 (e.g., downward), the free end moves away from the fixed end, and the elastic arm elastically deforms, that is, the fixed end, which is fixedly connected to the lower surface of the pressing structure, of the elastic arm moves toward the interior of the water tank housing 710 synchronously with the pressing structure, the elastic arm elastically deforms, and an included angle between the lower surface, to which the fixed end of the elastic arm is connected, of the pressing structure and the elastic arm decreases, so that a distance component between the free end and the fixed end of the elastic arm in the horizontal direction is increased; and after the external force is released, the free end moves toward the fixed end, and the elastic arm restores its shape, that is, the distance component between the free end and the fixed end of the elastic arm in the horizontal direction decreases. In some embodiments, the fixed end of the elastic arm is fixed in position, and the free end of the elastic arm may abut against other components (e.g., the water tank housing 710 or the pressing structure). During the movement of the free end of the elastic arm toward or away from the fixed end, the free end may slide relative to a surface of a component against which the free end abuts, that is, the free end moves from the first position to the second position, and the range of the space of movement between the first position and the second position is as described above and will not be repeated here. During sliding, the free end of the elastic arm may slide toward or away from the fixed end. For details, reference is made to the relevant descriptions of the elastic arm below. The elastic member 760 is configured such that: when the press button 750 is pressed toward the interior of the water tank housing 710 (e.g., downward), the elastic member 760 has a decrease in height and elastically deforms; and after the external force is released, the elastic member 760 has an increase in height and restores its shape. In some embodiments, during pressing the press button 750, the decrease in height of the elastic member 760 may be manifested as a lowered position of an upper end of the elastic member 760 (e.g., an end of the elastic member 760 connected to the press button 750), and in this case the height of a lower end of the elastic member 760 (e.g., the end of the elastic member 760 connected to the water tank housing 710) may remain unchanged. In some embodiments, the decrease in height of the elastic member 760 may also be manifested as a decrease in a vertical distance between the upper end and the lower end of the elastic member 760 in the height direction. Accordingly, the increase in height of the elastic member 760 may be manifested as rise in the position of the upper end of the elastic member (the height of the position of the lower end may remain unchanged) and an increase in the vertical distance between the upper end and the lower end of the elastic member 760 in the height direction.

In some embodiments, the fixed end of the elastic arm is fixedly connected to one of the side of the press button 750 close to the interior of the water tank housing 710 (e.g., the lower side) or the bottom wall of the recessed structure 720, and the free end of the elastic arm abuts against the other of the side of the press button 750 close to the interior of the water tank housing 710 (e.g., the lower side) or the bottom wall of the recessed structure 720. When the press button 750 is pressed downward under the action of an external force or after the external force is released, the free end slides relative to the other of the side (e.g., the lower side) of the press button 750 close to the interior of the water tank housing 710 or the bottom wall of the recessed structure 720. When the press button 750 is pressed toward the interior of the water tank housing 710 (e.g., downward) under the action of an external force, the free end slides away from the fixed end, and the elastic arm elastically deforms to accumulate elastic potential energy. After the external force is released, the free end slides toward the fixed end, and the elastic arm restores its shape to drive the press button 750 to resiliently return to its original position away from the interior of the water tank housing 710 (e.g., upward). It should be noted that, in this specification, the abutment refers to contact free of a force, or refers to contact and bearing a pre-pressure. As an example only, when the press button 750 is in a normal state (i.e., when not pressed by an external force), the free end abuts against the bottom wall of the recessed structure 720. This may mean that the free end just comes into contact with the bottom wall of the recessed structure 720 and is in an unstressed state, and the elastic arm does not elastically deform. Alternatively, the free end comes into contact with the bottom wall of the recessed structure 720, and the first free end 755-2 is in a stressed state, so that the elastic arm elastically deforms by a force, and the elastic arm accumulates elastic potential energy. In an embodiment in which the resilient structure is configured as an elastic arm, since the elastic arm includes a fixed end and a free end which are connected and transitioned by an arm body of a certain length, the elastic arm has a certain length and also has a certain length component in the horizontal direction. The elastic arm of a certain length enables the press button 750, especially the pressing structure connected to the elastic arm, to have a larger pressed stressed area, so that an effective pressure may be applied to the elastic arm within the range of the length component of the elastic arm in the horizontal direction, which increases the pressed stressed area of the press button 750 to facilitate the effective force application of the press button 750, thereby improving the stability and effectiveness of the use of the press button 750. In some embodiments, when the lower surface of the pressing structure is at a certain angle with the elastic arm (mainly the arm body of the elastic arm), the longer the arm body is, the larger the space of movement within which the press button 750 can move in the vertical direction, that is, the press button 750 may have an obvious space of movement in the vertical direction (e.g., upward and downward directions), thereby improving the sensitivity, stability and effectiveness of the press button 750 during use to ensure normal detachment or connection between the water tank and the main unit.

In some embodiments, when a force is applied to the middle region of the pressing structure, the stressed center of the elastic arm is also correspondingly located in the vicinity of the middle portion of the pressing structure, that is, a perpendicular at the stressed center of the elastic arm substantially coincides with a perpendicular at the middle portion of the pressing structure.

In some embodiments, the elastic arm may include at least one first elastic arm 755 and/or at least one second elastic arm 725. The first elastic arm 755 and/or the second elastic arm 725 may drive the press button 750 to resiliently return to its original position away from the interior of the water tank housing 710 (e.g., upward) after the press button 750 is pressed toward the interior of the water tank housing 710 (e.g., downward).

In some embodiments, the first elastic arm 755 is arranged on the side (e.g., the lower side) of the pressing surface 751 close to the interior of the water tank housing 710. The first elastic arm 755 includes a first fixed end 755-1 and a first free end 755-2, as shown in FIG. 1D. The first fixed end 755-1 of the first elastic arm 755 is fixed to a side of the press button 750 facing away from the pressing surface 751 (i.e., the side of the pressing surface 751 close to the interior of the water tank housing 710, e.g., the lower side), and the first free end 755-2 of the first elastic arm 755 abuts against the bottom wall of the recessed structure 720, as shown in FIG. 1B. In some embodiments, when the press button 750 is pressed toward the interior of the water tank housing 710 (e.g., downward) under the action of an external force, the first free end 755-2 of the first elastic arm 755 may slide relative to the bottom wall of the recessed structure 720, and the first elastic arm 755 deforms to accumulate elastic potential energy. In some embodiments, the first elastic arm 755 is arranged in the X direction. When the press button 750 is pressed toward the interior of the water tank housing 710 (e.g., downward) under the action of an external force, the first free end 755-2 of the first elastic arm 755 moves away from the first fixed end 755-1 in the X direction. Correspondingly, the first free end 755-2 of the first elastic arm 755 moves toward the first fixed end 755-1 in the Z direction. After the external force is released, the first elastic arm 755 releases the elastic potential energy, and the first free end 755-2 of the first elastic arm 755 slides on the bottom wall of the recessed structure 720, and the first free end 755-2 moves toward the first fixed end 755-1 in the X direction, and the first free end 755-2 moves away from the first fixed end 755-1 in the Z direction, so as to drive the press button 750 to move in the Z direction away from the interior of the water tank housing 710 (i.e., upward) to enable the press button 750 to return to the position before being pressed.

In some embodiments, when the press button 750 is in a normal state (i.e., when not pressed by an external force), the first free end 755-2 remains abutting against the bottom wall of the recessed structure 720, so that when the press button 750 is subsequently pressed by a force toward the interior of the water tank housing 710 (e.g., downward) and after the external force is released, the first elastic arm 755 may drive the press button 750 to return to the position before being pressed.

In some embodiments, the first free end 755-2 may be configured to be rounded, as shown in FIGS. 1C and 1D. The rounded configuration can reduce the frictions between the first free end 755-2 and the bottom wall of the recessed structure 720, thereby reducing the difficulty of the first free end 755-2 sliding relative to the bottom wall of the recessed structure 720.

In some embodiments, two or more first elastic arms 755 may be provided. Referring to FIG. 1D, in some embodiments, two first elastic arms 755 may be provided, and the two first elastic arms 755 are arranged in parallel and spaced apart in the Y direction, to ensure that the deformations of the two first elastic arms 755 can remain consistent during the process of the press button 750 being pressed and returning to its original position, thereby improving the stability and effectiveness of the press button 750.

Referring to FIG. 1F, in some embodiments, the second elastic arm 725 has similar or the same structure and function as the first elastic arm 755. For example, the second free end 725-2 of the second elastic arm 725 may also be rounded. In some embodiments, the second fixed end 725-1 of the second elastic arm 725 is fixed to the bottom wall of the recessed structure 720, and the second free end 725-2 of the second elastic arm 725 abuts against the side of the press button 750 away from the pressing surface 751 (i.e., the side of the press button 750 close to the interior of the water tank housing 710, i.e., the lower side). When the press button 750 is pressed toward the interior of the water tank housing 710 (e.g., downward) under the action of an external force, the second free end 725-2 of the second elastic arm 725 slides relative to the press button 750, and the second elastic arm 725 deforms to accumulate elastic potential energy. After the external force is released, the second free end 725-2 of the second elastic arm 725 slides relative to the lower side of the press button 750, and the second elastic arm 725 restores its shape and releases the elastic potential energy to drive the press button 750 to resiliently return to its original position away from the interior of the water tank housing 710 (e.g., upward). During the pressing and returning process of the press button 750, reference may be made to the relevant contents about the first elastic arm 755 for the working process and principle of the second elastic arm 725, which will not be repeated here.

In some embodiments, an orientation of the second elastic arm 725 may be parallel to an orientation of the first elastic arm 755, and in the X direction, the position of the first fixed end 755-1 of the first elastic arm 755 corresponds to the position of the second free end 725-2 of the second elastic arm 725, and the position of the first free end 755-1 of the first elastic arm 755 corresponds to the position of the second fixed end 725-1 of the second elastic arm 725. With the above arrangement, the forces applied by the second elastic arm 725 and the first elastic arm 755 to the press button 750 can counteract each other in the X direction, thereby improving the stability of the press button 750.

In some embodiments, two or more second elastic arms 725 may be provided. Referring to FIG. 1F, in some embodiments, two second elastic arms 725 may be provided, and the two second elastic arms 725 are arranged in parallel and spaced apart in the Y direction, to ensure that the deformations of the two first elastic arms 755 can remain consistent during the process of the press button 750 being pressed and returning to its original position, thereby improving the stability of the press button 750.

In some embodiments, the two second elastic arms 725 may be located between the two first elastic arms 755 in the Y direction. In some embodiments, the two first elastic arms 755 may be located between the two second elastic arms 725 in the Y direction. In some embodiments, the two first elastic arms 755 may be staggered with the two second elastic arms 725 in the Y direction.

In the above embodiment in which a plurality of elastic arms are provided, the plurality of elastic arms are preferably symmetrically distributed, the plurality of elastic arms may support the movement of the pressing structure in the vertical direction at different positions during the movement of the pressing structure of the press button 750, thereby improving the stability and smoothness of the press button 750 during use. Moreover, the arrangement of the plurality of elastic arms at different positions expands the stressed area of the resilient structure, thus increasing the pressed stressed area of the pressing structure of the press button 750 to facilitate the vertical (e.g., downward) movement of the pressing structure in different regions of the pressing surface 751, and thus improving the stability, smoothness, effectiveness and usability of the press button 750 during use. In addition, since the plurality of elastic arms are provided, the resilient structure can still work when one or more of the elastic arms are damaged, so that the press button 750 can work normally, thereby ensuring normal detachment or connection between the water tank and the main unit.

Referring to FIG. 1B, in some embodiments, one end of the elastic member 760 may be fixedly connected to one of the lower side of the pressing surface 751 or the bottom wall of the recessed structure 720, that is, one end of the elastic member 760 is fixed, and the other end may abut against the other of the side of the press button 750 facing away from the pressing surface 751 (i.e., the lower side of the pressing surface 751) or the bottom wall of the recessed structure 720. That is, one end of the elastic member 760 may be fixed to the lower side of the pressing surface 751, and the other end may abut against the bottom wall of the recessed structure 720; Alternatively, one end of the elastic member 760 may be fixed to the bottom wall of the recessed structure 720, and the other end may abut against the lower side of the pressing surface 751. When the press button 750 is pressed toward the interior of the water tank housing 710 (e.g., downward) under the action of an external force, the elastic member 760 has a decrease in height and elastically deforms to accumulate elastic potential energy. After the external force is released, the elastic member 760 begins to restore its shape and drives the press button 750 to move away from the interior of the water tank housing 710 (e.g., upward) to return to the position before being pressed.

In some embodiments, one end of the elastic member 760 may also be connected to one of the lower side of the pressing surface 751 or the bottom wall of the recessed structure 720 in a plug-in manner. Specifically, referring to FIGS. 1B and 1D, a connecting protrusion 756 may be provided on the side of the press button 750 facing away from the pressing surface 751 (i.e., the lower side of the press button 750) or the bottom wall of the recessed structure 720, and the connecting protrusion 756 may be connected to the elastic member 760. When the connecting protrusion 756 is inserted into the elastic member 760, the elastic member 760 is mounted and fixed by means of the connecting protrusion 756. When the connecting protrusion 756 is pulled out from the elastic member 760, the elastic member 760 can be detached and replaced.

In some embodiments, the elastic member 760 may be arranged in the middle position between the recessed structure 720 and the press button 750 in the X direction (as shown in FIGS. 1B and 1D). On the basis that the pressing region on the press button 750 that is acted upon by an external force is located in the middle region of the pressing body (pressing structure) in the length direction, when a force is applied to the middle region of the pressing structure of the pressing body, the stressed center of the elastic member 760 is also correspondingly located in the vicinity of the middle portion of the pressing structure, that is, a perpendicular at the stressed center of the elastic member substantially coincides with a perpendicular at the middle portion of the pressing structure, so as to prevent the press button 750 from tilting in the X direction, thereby improving the stability of the press button 750. In this case, in the X direction, the elastic member 760 may be located between the connecting structure 752 and the first free end 755-2 of the first elastic arm 755, as shown in FIGS. 1B and 1D. In some embodiments, the elastic member 760 may be arranged at the middle position between the recessed structure 720 and the press button 750 in the Y direction (as shown in FIG. 1D), the two first elastic arms 755 may be respectively arranged on the two sides of the elastic member 760, and the two second elastic arms 725 may be respectively arranged on the two sides of the elastic member 760, thereby improving the stability of the press button 750.

In some embodiments, the elastic member 760 may include an elastic material (e.g., silicone) or an elastic structure (e.g., a spring). In some embodiments, the elastic member 760 may be a spring which has a long service life and is easy to replace.

In some embodiments, either an elastic member 760 or a second elastic arm 725 may be provided in the recessed structure 720. In some embodiments, both the elastic member 760 and the second elastic arm 725 may be provided in the recessed structure 720.

FIG. 1G is another exemplary structural schematic diagram of the press button according to some embodiments of this specification. Referring to FIG. 1G, in some embodiments, the press button 750 may further include a fixing structure 757, and the resilient structure may include an elastic piece mechanism 758. The fixing structure 757 may be arranged on a peripheral side of the press button 750 except the side on which the connecting structure 752 is provided, and the press button 750 is connected to the water tank housing 710 by means of the fixing structure 757. In the X direction, the fixed end of the elastic piece mechanism 758 is connected to the fixing structure 757, and a connecting structure 752 is provided at the free end of the elastic piece mechanism 758; and in the Y direction, gaps are respectively provided between the two sides of the elastic piece mechanism 758 and the fixing structure 757, so that the free end of the elastic piece mechanism 758 can move relative to the fixed end of the elastic piece mechanism 758.

In some embodiments, the pressing surface 751 is arranged on a surface (i.e., an upper surface) of the elastic piece mechanism 758 that is away from the interior of the water tank housing 710.

In some embodiments, when the press button 750 is pressed toward the interior of the water tank housing 710 (e.g., downward), the free end of the elastic piece mechanism 758 moves toward the interior of the water tank housing 710, and the elastic piece mechanism 758 elastically deforms. After the external force is released, the free end of the elastic piece mechanism 758 moves away from the interior of the water tank housing 710, and the elastic piece mechanism 758 restores its shape to drive the press button 750 to resiliently return to its original position away from the interior of the water tank housing 710.

In some embodiments, at least one of the elastic arm (e.g., the first elastic arm 755 and/or the second elastic arm 725) and the elastic member 760 may also be provided between the elastic piece mechanism 758 and the water tank housing 710. The elastic arm and/or the elastic member are arranged on the side of the elastic piece mechanism 758 facing the interior of the water tank housing 710 (e.g., the lower surface of the elastic piece mechanism 758), or are arranged on the surface of the recessed structure 720 of the water tank housing 710 corresponding to the press button 750, which will not be limited in this specification.

Some embodiments of this specification further provide a ventilation therapy device, including the water tank 700 described above. In some embodiments, the ventilation therapy device may include a ventilator device and a high-flow respiratory humidification therapy device, etc.

In the above embodiments, the arrangement of the press button enables the water tank to be detachably connected to the main unit, which reduces the operation difficulty of the detachment of the water tank and allows the water tank to be connected to the main unit in a flexible manner. The arrangement of the resilient structure on the press button enables the press button to automatically return to its original position after being pressed, which does not require additional operations and has simple steps. The movable arrangement of the press button relative to the water tank housing enables the water tank to remain stable during the detachment process and be not prone to shaking relative to the main unit, which not only increases the diversity of connection between the water tank and the main unit, but also reduces the difficulty of connecting the water tank to the main unit, making it easier for the mounting and detachment of the water tank.

It should be noted that different embodiments may produce different beneficial effects. In different embodiments, the beneficial effects that may be produced may be any one or a combination of the above beneficial effects, or any other possible beneficial effects.

The basic concepts have been described above, and it will be apparent to those skilled in the art that the foregoing detailed disclosure is intended to be exemplary only and does not constitute a limitation to this specification. Although not explicitly stated herein, those skilled in the art may make various modifications, improvements and amendments to this specification. Such modifications, improvements and amendments are suggested in this specification, and thus remain within the spirit and scope of the exemplary embodiments of this specification.

Meanwhile, this specification uses specific words to describe the embodiments of this specification. For example, "one embodiment", "an embodiment" and/or "some embodiments" mean a particular feature, structure or characteristic related to at least one embodiment of this specification. Hence, it should be emphasized and noted that "an embodiment" or "one embodiment" or "one alternative embodiment" mentioned in two or more different positions in this specification does not necessarily refer to the same embodiment. Furthermore, some features, structures or characteristics in one or more embodiments of this specification may be appropriately combined.

Similarly, it should be noted that in order to simplify the presentation of the disclosure of this specification, and thereby help to understand one or more embodiments of the invention, in the previous descriptions of the embodiments of this specification, various features are sometimes combined into one embodiment, the accompanying drawings, or descriptions thereof. However, this method of disclosure does not imply that the subject of this specification requires more features than those mentioned in the claims. In fact, the embodiments have fewer features than all of the individual embodiments disclosed above.

Finally, it should be understood that the embodiments described in this specification are only intended to illustrate the principles of the embodiments of this specification. Other variations may also fall within the scope of this specification. Thus, by way of example but not limitation, alternative configurations of the embodiments of this specification can be considered consistent with the teachings of this specification. Accordingly, the embodiments of this specification are not limited to those explicitly presented and described in this specification.

## Claims

1. A press button for mounting and detachment of a water tank from a main unit of a ventilation therapy device, **characterized in that** the press button is arranged on the water tank, and is configured to be pressed toward an interior of a water tank housing of the water tank under the action of an external force such that the water tank is mounted to or detached from the main unit; wherein
the press button comprises a pressing body, the pressing body comprising a resilient structure, the resilient structure comprising a fixed end and a free end arranged opposite the fixed end, and the resilient structure being configured such that: when the press button is pressed toward the interior of the water tank housing, the free end moves from a first position to a second position, and the resilient structure elastically deforms; and after the external force is released, the free end moves from the second position to the first position, and the resilient structure restores its shape to drive the press button to resiliently return to its original position away from the interior of the water tank housing such that the water tank is connected to the main unit or returns to its original position after detachment.

2. The press button according to claim 1, **characterized in that** the press button further comprises a connecting structure, wherein the connecting structure is arranged at an end of the pressing body close to the main unit, the connecting structure is detachably connected to the main unit, and when the pressing body is pressed toward the interior of the water tank housing, the connecting structure moves toward the interior of the water tank housing and the connecting structure is separated from the main unit.

3. The press button according to claim 2, **characterized in that** the pressing body comprises a pressing structure, and when the pressing structure is pressed toward the interior of the water tank housing, the connecting structure moves toward the interior of the water tank housing such that the connecting structure is disengaged from an engagement portion of the main unit located above the connecting structure.

4. The press button according to claim 3, **characterized in that** the connecting structure comprises a connecting member and a snap-fit protrusion, wherein the connecting member is connected to an end of the pressing body, the snap-fit protrusion is arranged at an end of the connecting member away from the pressing body, and the snap-fit protrusion is configured to be engaged with or separated from the engagement portion of the main unit; and the connecting member has a preset length, an inner surface of a housing of the main unit provides the engagement portion, the engagement portion is positioned corresponding to the preset length of the connecting member, and a groove that is engaged with the snap-fit protrusion is provided at the engagement portion.

5. The press button according to claim 4, **characterized in that** the preset length of the connecting member ranges from 5 mm to 25 mm.

6. The press button according to claim 3, **characterized in that** the pressing structure is arranged between the free end and the fixed end of the resilient structure.

7. The press button according to claim 1, **characterized in that** a pressing region of the press button that is acted upon by the external force is located in a middle region of the pressing body in a length direction.

8. The press button according to claim 3, **characterized in that** the free end of the resilient structure abuts against one of a surface of the water tank housing or a surface of the pressing structure, and the fixed end is fixedly connected to the other of the surface of the water tank housing or the surface of the pressing structure.

9. The press button according to claim 7, **characterized in that** a stressed center of the resilient structure corresponds to the middle region of the pressing body in the length direction.

10. The press button according to any one of claims 1 to 5 and 7 to 9, **characterized in that** the resilient structure comprises at least one of an elastic arm and an elastic member;
the elastic arm is configured such that: when the press button is pressed toward the interior of the water tank housing, the free end moves away from the fixed end from the first position to the second position, and the elastic arm elastically deforms; and after the external force is released, the free end moves toward the fixed end from the second position to the first position, and the elastic arm restores its shape; and
the elastic member is configured such that: when the press button is pressed toward the interior of the water tank housing, the free end moves from the first position to the second position, and the elastic member has a decrease in height and elastically deforms; and after the external force is released, the free end moves from the second position to the first position, and the elastic member has an increase in height and restores its shape.

11. The press button according to claim 10, **characterized in that** the fixed end of the elastic arm is fixedly connected to one of a side of the press button close to the interior of the water tank housing or an outer surface of the water tank housing, and the free end of the elastic arm abuts against the other of the side of the press button close to the interior of the water tank housing or the outer surface of the water tank housing, and when the press button is pressed toward the interior of the water tank housing or after the external force is released, the free end slides relative to the other of the side of the press button close to the interior of the water tank housing or the outer surface of the water tank housing.

12. The press button according to claim 11, **characterized in that** the elastic arm comprises at least one first elastic arm and/or at least one second elastic arm,
a first fixed end of the first elastic arm is fixed to the side of the press button close to the interior of the water tank housing, and a first free end of the first elastic arm abuts against the outer surface of the water tank housing, and when the press button is pressed toward the interior of the water tank housing or the external force is released, and the first free end slides relative to an upper surface of the water tank housing; and
a second fixed end of the second elastic arm is fixed to the outer surface of the water tank housing, and a second free end of the second elastic arm abuts against the side of the press button close to the interior of the water tank housing, and when the press button is pressed toward the interior of the water tank housing or after the external force is released, the second free end slides relative to the side of the press button close to the interior of the water tank housing.

13. The press button according to claim 10, **characterized in that** the fixed end of the elastic member is fixedly connected to one of the side of the press button close to the interior of the water tank housing or the outer surface of the water tank housing, and the free end of the elastic member abuts against the other of the side of the press button close to the interior of the water tank housing or the outer surface of the water tank housing.

14. The press button according to claim 13, **characterized in that** a connecting protrusion is provided on one of the side of the press button close to the interior of the water tank housing or the outer surface of the water tank housing, and the connecting protrusion is connected to the elastic member.

15. The press button according to any one of claims 1 to 5 and 7 to 9, **characterized in that** at least one of a limiting structure and a guide structure is further provided between the press button and the outer surface of the water tank housing, wherein
the limiting structure is configured to limit a range of movement of the press button away from the interior of the water tank housing; and
the guide structure is configured to guide the press button to move along a preset path.

16. The press button according to claim 15, **characterized in that** the limiting structure comprises a limiting member and a limiting slot, wherein the limiting member is arranged on one of the side of the press button close to the interior of the water tank housing or the outer surface of the water tank housing, the limiting slot is arranged on the other of the side of the press button close to the interior of the water tank housing or the outer surface of the water tank housing, and the limiting member is located in the limiting slot and is movable in the limiting slot toward and/or away from the interior of the water tank housing.

17. The press button according to claim 15, **characterized in that** the guide structure comprises a guide member and a guide groove, wherein the guide member is arranged on one of the side of the press button close to the interior of the water tank housing or the outer surface of the water tank housing, the guide groove is arranged on the other of the side of the press button close to the interior of the water tank housing or the outer surface of the water tank housing, and the guide member is movable in the guide groove toward and/or away from the interior of the water tank housing.

18. A water tank, **characterized by** comprising a press button according to any one of claims 1 to 17.

19. The water tank according to claim 18, **characterized in that** the outer surface of the water tank housing comprises a recessed structure, the press button is received in the recessed structure, and the resilient structure is arranged between the side of the press button close to the interior of the water tank housing and a bottom wall of the recessed structure.

20. A ventilation therapy device, **characterized by** comprising a water tank according to any one of claims 18 and 19.

21. A press button for mounting and detachment of a water tank from a main unit of a ventilation therapy device, **characterized in that** the press button is arranged on the water tank, and is configured to be pressed toward an interior of a water tank housing of the water tank under the action of an external force such that the water tank is mounted to or detached from the main unit; wherein
the press button comprises a pressing body, the pressing body comprising a resilient structure, the resilient structure comprising an elastic arm, the elastic arm comprising a free end and a fixed end arranged opposite the free end, and the elastic arm being configured such that: when the press button is pressed toward the interior of the water tank housing, the free end slides away from the fixed end, and the elastic arm elastically deforms; and after the external force is released, the free end slides toward the fixed end, and the elastic arm restores its shape such that the water tank is connected to the main unit or returns to its original position after detachment.

22. The press button according to claim 21, **characterized in that** the press button further comprises a connecting structure, wherein the connecting structure is arranged at an end of the pressing body close to the main unit, the connecting structure is detachably connected to the main unit, and when the pressing body is pressed toward the interior of the water tank housing, the connecting structure moves toward the interior of the water tank housing and the connecting structure is separated from the main unit.

23. The press button according to claim 22, **characterized in that** the pressing body comprises a pressing structure, and when the pressing structure is pressed toward the interior of the water tank housing, the connecting structure moves toward the interior of the water tank housing such that the connecting structure is disengaged from an engagement portion of the main unit located above the connecting structure.

24. The press button according to claim 23, **characterized in that** the connecting structure comprises a connecting member and a snap-fit protrusion, wherein the connecting member is connected to an end of the pressing body, the snap-fit protrusion is arranged at an end of the connecting member away from the pressing body, and the snap-fit protrusion is configured to be engaged with or separated from the engagement portion of the main unit; and the connecting member has a preset length, an inner surface of a housing of the main unit provides the engagement portion, the engagement portion is positioned corresponding to the preset length of the connecting member, and a groove that is engaged with the snap-fit protrusion is provided at the engagement portion.

25. The press button according to claim 24, **characterized in that** the preset length of the connecting member ranges from 5 mm to 25 mm.

26. The press button according to claim 21, **characterized in that** a pressing region of the press button that is acted upon by the external force is located in a middle region of the pressing body in a length direction.

27. The press button according to claim 26, **characterized in that** a stressed center of the elastic arm corresponds to the middle region of the pressing body in the length direction.

28. The press button according to any one of claims 21 to 27, **characterized in that** the fixed end of the elastic arm is fixedly connected to one of a side of the press button close to the interior of the water tank housing or an outer surface of the water tank housing, and the free end of the elastic arm abuts against the other of the side of the press button close to the interior of the water tank housing or the outer surface of the water tank housing, and when the press button is pressed toward the interior of the water tank housing or after the external force is released, the free end slides relative to the other of the side of the press button close to the interior of the water tank housing or the outer surface of the water tank housing.

29. The press button according to claim 28, **characterized in that** the elastic arm comprises at least one first elastic arm and/or at least one second elastic arm,
a first fixed end of the first elastic arm is fixed to the side of the press button close to the interior of the water tank housing, and a first free end of the first elastic arm abuts against the outer surface of the water tank housing, and when the press button is pressed toward the interior of the water tank housing or the external force is released, and the first free end slides relative to an upper surface of the water tank housing; and
a second fixed end of the second elastic arm is fixed to the outer surface of the water tank housing, and a second free end of the second elastic arm abuts against the side of the press button close to the interior of the water tank housing, and when the press button is pressed toward the interior of the water tank housing or after the external force is released, the second free end slides relative to the side of the press button close to the interior of the water tank housing.

30. The press button according to any one of claims 21 to 27, **characterized in that** at least one of a limiting structure and a guide structure is further provided between the press button and the outer surface of the water tank housing, wherein
the limiting structure is configured to limit a range of movement of the press button away from the interior of the water tank housing; and
the guide structure is configured to guide the press button to move along a preset path.

31. The press button according to claim 30, **characterized in that** the limiting structure comprises a limiting member and a limiting slot, wherein the limiting member is arranged on one of the side of the press button close to the interior of the water tank housing or the outer surface of the water tank housing, the limiting slot is arranged on the other of the side of the press button close to the interior of the water tank housing or the outer surface of the water tank housing, and the limiting member is located in the limiting slot and is movable in the limiting slot toward and/or away from the interior of the water tank housing.

32. The press button according to claim 30, **characterized in that** the guide structure comprises a guide member and a guide groove, wherein the guide member is arranged on one of the side of the press button close to the interior of the water tank housing or the outer surface of the water tank housing, the guide groove is arranged on the other of the side of the press button close to the interior of the water tank housing or the outer surface of the water tank housing, and the guide member is movable in the guide groove toward and/or away from the interior of the water tank housing.

33. A water tank, **characterized by** comprising a press button according to any one of claims 21 to 32.

34. The water tank according to claim 33, **characterized in that** the outer surface of the water tank housing comprises a recessed structure, the press button is received in the recessed structure, and the resilient structure is arranged between the side of the press button close to the interior of the water tank housing and a bottom wall of the recessed structure.

35. A ventilation therapy device, **characterized by** comprising a water tank according to any one of claims 33 and 34.

36. A press button for mounting and detachment of a water tank from a main unit of a ventilation therapy device, **characterized in that** the press button is arranged on the water tank, and is configured to be pressed toward an interior of a water tank housing of the water tank under the action of an external force such that the water tank is mounted to or detached from the main unit; wherein
the press button comprises a pressing body, the pressing body comprising a resilient structure, the resilient structure comprising an elastic member, and the elastic member being configured such that: when the press button is pressed toward the interior of the water tank housing, the elastic member has a decrease in height and elastically deforms; and after the external force is released, the elastic member has an increase in height and restores its shape such that the water tank is connected to the main unit or returns to its original position after detachment.

37. The press button according to claim 36, **characterized in that** the press button further comprises a connecting structure, wherein the connecting structure is arranged at an end of the pressing body close to the main unit, the connecting structure is detachably connected to the main unit, and when the pressing body is pressed toward the interior of the water tank housing, the elastic member is compressed, the connecting structure moves toward the interior of the water tank housing and the connecting structure to move in the pressing direction until the connecting structure is separated from the main unit.

38. The press button according to claim 37, **characterized in that** the pressing body comprises a pressing structure, and when the pressing structure is pressed toward the interior of the water tank housing, the connecting structure moves toward the interior of the water tank housing such that the connecting structure is disengaged from an engagement portion of the main unit located above the connecting structure.

39. The press button according to claim 38, **characterized in that** the connecting structure comprises a connecting member and a snap-fit protrusion, wherein the connecting member is connected to an end of the pressing body, the snap-fit protrusion is arranged at an end of the connecting member away from the pressing body, and the snap-fit protrusion is configured to be engaged with or separated from the engagement portion of the main unit; and the connecting member has a preset length, an inner surface of a housing of the main unit provides the engagement portion, the engagement portion is positioned corresponding to the preset length of the connecting member, and a groove that is engaged with the snap-fit protrusion is provided at the engagement portion.

40. The press button according to claim 39, **characterized in that** the preset length of the connecting member ranges from 5 mm to 25 mm.

41. The press button according to claim 36, **characterized in that** a pressing region of the press button that is acted upon by the external force is located in a middle region of the pressing body in a length direction.

42. The press button according to claim 41, **characterized in that** a stressed center of the elastic member corresponds to the middle region of the pressing body in the length direction.

43. The press button according to any one of claims 36 to 42, **characterized in that** one end of the elastic member is fixedly connected to one of the side of the press button close to the interior of the water tank housing or the outer surface of the water tank housing, and the other end of the elastic member abuts against the other of the side of the press button close to the interior of the water tank housing or the outer surface of the water tank housing.

44. The press button according to claim 43, **characterized in that** the one end of the elastic member is connected to one of the side of the press button close to the interior of the water tank housing or the outer surface of the water tank housing in a plug-in manner.

45. The press button according to claim 44, **characterized in that** a connecting protrusion is provided on one of the side of the press button close to the interior of the water tank housing or the outer surface of the water tank housing, and the connecting protrusion is connected to the elastic member.

46. The press button according to any one of claims 36 to 42, **characterized in that** at least one of a limiting structure and a guide structure is further provided between the press button and the outer surface of the water tank housing, wherein
the limiting structure is configured to limit a range of movement of the press button away from the interior of the water tank housing; and
the guide structure is configured to guide the press button to move along a preset path.

47. The press button according to claim 46, **characterized in that** the limiting structure comprises a limiting member and a limiting slot, wherein the limiting member is arranged on one of the side of the press button close to the interior of the water tank housing or the outer surface of the water tank housing, the limiting slot is arranged on the other of the side of the press button close to the interior of the water tank housing or the outer surface of the water tank housing, and the limiting member is located in the limiting slot and is movable in the limiting slot toward and/or away from the interior of the water tank housing.

48. The press button according to claim 46, **characterized in that** the guide structure comprises a guide member and a guide groove, wherein the guide member is arranged on one of the side of the press button close to the interior of the water tank housing or the outer surface of the water tank housing, the guide groove is arranged on the other of the side of the press button close to the interior of the water tank housing or the outer surface of the water tank housing, and the guide member is movable in the guide groove toward and/or away from the interior of the water tank housing.

49. A water tank, **characterized by** comprising a press button according to any one of claims 36 to 48.

50. The water tank according to claim 49, **characterized in that** the outer surface of the water tank housing comprises a recessed structure, the press button is received in the recessed structure, and the resilient structure is arranged between the side of the press button close to the interior of the water tank housing and a bottom wall of the recessed structure.

51. A ventilation therapy device, **characterized by** comprising a water tank according to any one of claims 49 and 50.
